# EUROPEAN PATENT SPECIFICATION

(11) **EP 0 894 145 B1**
(45) Date of publication and mention of the grant of the patent: **21.09.2005**
(21) Application number: 97905316.2
(22) Date of filing: 03.03.1997
(51) Int. Cl.: C12Q 1/68

(54) **DRUG TRIAL ASSAY SYSTEM**
DROGEN UNTERSUCHUNGSSYSTEM
SYSTEME DE DOSAGE POUR DES ESSAIS DE MEDICAMENTS

(30) Priority: 01.03.1996 GB 9604480; 16.03.1996 GB 9605598
(43) Date of publication of application: 03.02.1999
(73) Proprietor: THE UNIVERSITY COURT OF THE UNIVERSITY OF DUNDEE, Scotland (GB)
(72) Inventor: BURCHELL, Brian, Tayport, Fife DD6 9 JB (GB)
(74) Representative: MacDougall, Donald Carmichael
(86) International application number: PCT/GB1997/000577
(87) International publication number: WO 1997/032042

(56) References cited:
- WO-A-92/12987
- N. ENGL. J. MED, vol. 333, no. 18, November 1995, pages 1171-5, XP002040437 BOSMA P ET AL: "The genetic basis of reduced expression of bilirubin UDP glucuronsyltransferase 1 in Gilbert's syndrome"
- PHARMACOKINETICS, vol. 2, no. 3, 1992, pages 93-108, XP002040438 OWENS I ET AL: "The novel bilirubin/phenol UDP-glucuronosyltransferase UGT1 gene locus: implications for multiple familial hyperrubinemia phenotypes "
- THE LANCET, vol. 347, 2 March 1996, pages 578-81, XP002040439 MONAGHAN G ET AL: "Genetic variation in bilirubin UDP-glucuronosyltransferase gene promoter and Gilbert's syndrome" cited in the application
- GASTROENTEROLOGY, vol. 102, January 1992, pages 577-86, XP002040440 DE MORAIS S ET AL: "Decreased glucuronidation and increased bioactivation of acetaminophen in Gilbert's syndrome" cited in the application
- MOLECULAR PHARMACOLOGY, vol. 43, no. 4, April 1993, pages 649-54, XP002040441 EBNER, T ET AL: "Human bilirubin UDP-gluconosyltransferase catalyzes the glucoronidation of ethinylestradiol" cited in the application

## Description

The present invention relates to drug trials, usually carried out for or on behalf of pharmaceutical companies. More particularly the invention relates to a method for improving the efficacy of drug trials.

In the different stages of drug trials, regulatory authorities in different European countries and the FDA in the USA require extensive data to be provided in order to approve use of the drugs.

It is important that as much information as possible is available in relation to all participants who take part in drug trials, from volunteers who take part in phase 1 trials to patients involved in stage 3 clinical trials.

In particular, if certain individuals or groups of individuals have severe or abnormal reactions to drug administration, further studies involving that drug will be in jeopardy unless the reason for the reaction is realised.

The knowledge of pharmacogenetics can play an important role in understanding the impact of drug metabolism on pharmacokinetics, role of receptor variants in drug response and in the selection of patient populations for clinical studies.

Considerable effort has been expended in attempting to identify the pharmacogenetic basis of idiosyncsatic adverse drug reactions, particularly hypersensitivity reactions. While there is clear evidence for pharmacogenetic influence on susceptibility to hypersensitivity reactions, necessary and sufficient pharamacogenetic defects have not been identified.

The clinical implications of genetic polymorphism in drug metabolism have been studied extensively (See Tucker GT (1994) Journal Pharamacology 46 pages 417-424).

Gilbert's Syndrome (GS) is a benign unconjugated hyperbilirubinaemia occurring in the absence of structural liver disease and overt haemolysis and characterized by episodes of mild intermittent jaundice. It is part of a spectrum of familial unconjugated hyperbilirubinaemias including the more severe Crigler-Najjar (CN) syndromes (types 1 and 2). GS is the most common inherited disorder of hepatic bilirubin metabolism occurring in 2-12% of the population and is often detected in adulthood through routine screening blood tests or the fasting associated with surgery/intercurrent illness which unmasks the hyperbilirubinaemia¹⁻³. The most consistent feature in GS is a deficiency in bilirubin glucuronidation but altered metabolism of drugs has also been reported³⁻⁵. Altered rates of bilirubin production, hepatic haem production and altered hepatic uptake of bilirubin have been reported in some GS patients².

Due to the benign nature of the syndrome and its prevalence in the population it may be more appropriate to consider GS as a normal genetic varriant² exhibiting a reduced bilirubin glucuronidation capacity (which in certain situations such as fasting, illness or administration of drugs) could precipitate jaundice.

In drug trials where high levels of serum total bilirubin is detected for certain individuals, it is not clear whether this is because the individuals have Gilbert's Syndrome or if it is because of an effect of the drug. Whereas presently, results are explained merely by saying that the individuals have Gilbert's Syndrome, it is suspected that in the future, it will be necessary to prove this fact.

Where a jaundiced phenotype is apparent after volunteers have been accepted for a trial and have been subjected to five days of a strict diet, no alcohol and no smoking the jaundiced appearance giving an indication that the individuals have Gilbert's Syndrome, may cause them to be ruled out of the trials. Therefore, where approximately 250 individuals would be required for phase 1 trials and about 6000 patients for phase 3 trials, unnecessary time and effort would have been spent during the first 5 days of these trials and individuals having Gilbert's Syndrome may be ill effected.

The present invention aims to provide a method of improving the efficacy of drug trials in view of the problems mentioned above.

According to the present invention there is provided a method of conducting a drug trial, the method comprising screening samples from potential participants for the basis of Gilbert's Syndrome and eliminating or including potential participants in a drug trial in the knowledge of them possessing or not possessing the genetic basis of Gilbert's Syndrome, and interpreting hyperbilirubinaemia and/or jaundice occurring during the drug trial in the knowledge that certain participants have or do not have Gilbert's Syndrome.

In a preferred embodiment of the invention the method comprises the steps taking a sample from each potential participant in a drug trial, screening the samples for the genetic basis of Gilbert's Syndrome, identifying participants having the genetic basis of Gilbert's Syndrome, proceeding with the drug trial in the knowledge of participants possessing or not possessing the genetic basis of Gilbert's syndrome; and interpreting hyperbilirubinaemia and/or jaundice occurring during the drug trial in the knowledge that certain participants have or do not have Gilbert's Syndrome.

The sample may comprise blood, a buccal smear or any other sample containing DNA from the individual to be tested.

In one embodiment the method comprises the further step of eliminating participants having the genetic basis of Gilbert's Syndrome from the drug trial.

Preferably the method comprises the steps of isolating DNA from each sample, amplifying the DNA in a region indicating the genetic basis of Gilbert's Syndrome, isolating amplified DNA fragments by gel electrophoresis and identifying individuals having the genetic basis of Gilbert's disease.

Preferably the DNA is amplified using the polymerase chain reaction (PCR) using a radioactively labelled pair of nucleotide primers.

The primers are designed to prime the amplification reaction at either side of an area of the genome known to be associated with Gilbert's Syndrome.

Preferably the DNA region indicating the genetic basis of Gilbert's Syndrome is the gene encoding UDP-glucuronosyltransferase (UGT).

By gene is meant, the non coding and coding regions and the upstream and downstream noncoding regions.

In a preferred embodiment the DNA to be amplified is in an upstream promoter region of the UGT1*1 exon1.

Most preferably the DNA to be amplified includes the region between -35 and -55 nucleotides at the 5' end of UGT1*1 exon.

According to the invention there are provided suitable primers for use in a PCR reaction including primer pairs; A/B(A,5'-AAGTGAACTCCCTGCTACCTT-3', B,5'-CCACTGGGATCAACAGTATCT-3') or

C/D (C,5'-GTCACGTGACACAGTCAAAC-3';

D 5'-TTTGCTCCTGCCAGAGGTT-3')

The invention further comprises a kit for screeing individuals for participation in drug trials, the kit comprising primers for amplifying DNA in a region of the genome indicating the genetic basis of Gilbert's Syndrome.

Using primer sequences as described herein, DNA can be amplified and analysed using among others any of the following protocols;

### Protocol 1 Radioactive method

1. Extract DNA from Buccal Cells or 3ml Blood.
2. Choose primers from either side of the "TATA" box region of UGT1*1 exon1 regulatory sequence. Freshly end label one primer with [γ ³²α]-ATP (40 min).
3. Amplifying a small region up to 100 bp in length by PCR (2h).
4. Apply to 6% PAG denaturing gel (preparation, loading, run time, 4h).
5. Expose (-70°C) wet gel to autoradiographic film (15 min).

This method takes about 7h to complete. Polymorphisms only observed in TATA box non coding region todate.

### Protocol 2

### Alternative Radioactive Method: Solid Phase

### Minisequencing

1. Extract DNA (as above)
2. Prepare primers biotinylating one
3. Amplify DNA by PCR using primers
4. Captive biotinylated PCR products on streptavidin coated support and deactive.
5. Carry out primer extension reaction sequencing.

### Protocol 3

### Non-Radioactive Methods:

### (a) Analysis by Single Strand Conformational Polymorphism (SSCP)

1. Extract DNA (as above).
2. Choose primers either side of the TATA Box.
3. Amplify a small region up to 100 bp in length by PCR (2H).
4. Denature and place on ice (15 min).
5. Load onto a non-denaturing PAG gel, (preparation/load/run time, 4h).
6. Stain with Ethidium bromide or silver nitrate (30 mm).

This method still takes about 7h to complete, but is potentially slightly cheaper since there is no radioactivity or autoradiography.

This method could be done on an automated DNA sequencer from stage 5, if primers are tagged with chromophores in PCR stages 2 and 3. Result would then be read automatically.

### (b) Oligonucleotide Assay Hybridization

1. Extract DNA (as above).
2. Choose primers and amplify DNA by PCR up to 100 bp in length.
3. Apply DNA to plastic grids.
4. Screen bound DNA samples with specific DNA probes for TA₅, TA₆, TA₇ tagged with different coloured/fluorescent chromphores.
5. Read output automatically for experimental protocols.

### References

Monaghan G et al. Lancet (1996) 347 578-581.

"Detection of polymorphisms of human DNA by gel electrophoresis or single-stranded conformational polymorphisms". Orita M et al. Proc Matl Acad Sci (USA) (1989) 86 2766-2700.

"Assays of complementary oligonucleotides for analysing Hybridization behaviour of Nucleic Acids". Southern E M. Nuc Acids Res (1194) 22 1368-1373.

In a further aspect there is provided a method of conducting a drug trial, the method comprising screening a sample from each participant of a drug trial for the genetic basis of Gilbert's Syndrome and identifying participants having the genetic basis of Gilbert's Syndrome, carrying out said drug trial and detecting serum total bilirubin such that it is possible to detect whether high levels of serum total bilirubin in a participant of said drug trial is caused by the drug or Gilbert's Syndrome.

The basis of the invention is illustrated in the following example with reference to the accompanying figures wherein:
Figure 1 illustrates genotypes at the TATA box sequence upstream of the UGT1*1 exon 1 determined by direct sequencing and radioactive PCR.
Figure 2 illustrates serum total bilirubin (µmol/l) plotted against UGT1*1 exon 1 genotype.
Figure 3 illustrates segregation of the 7/7 genotype with elevated serum total bilirubin concentration in a family with GS.

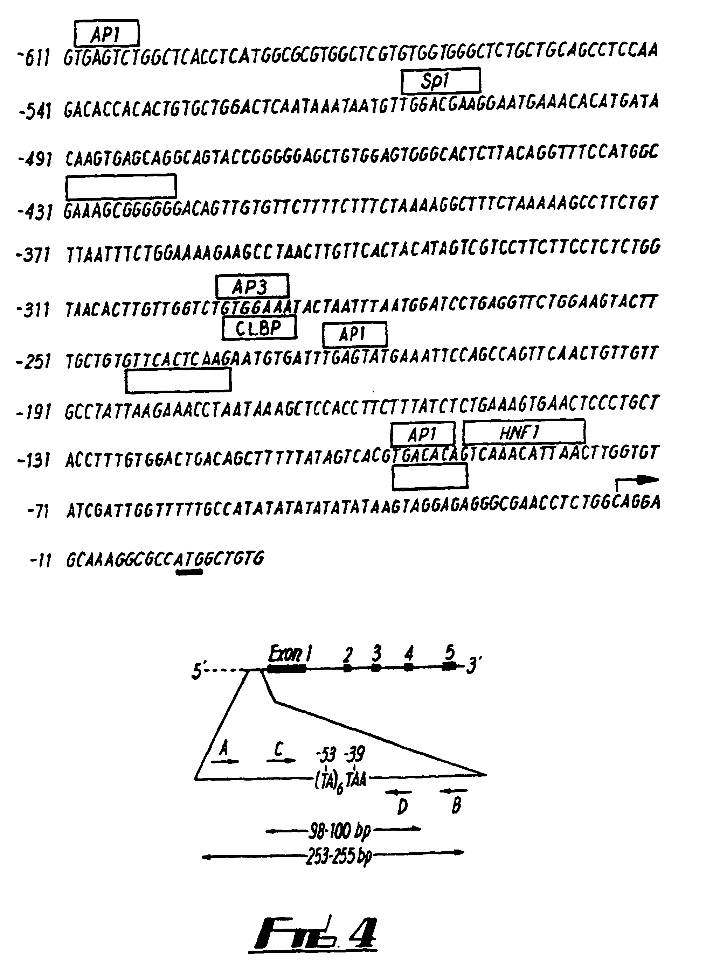

Figure 4 illustrates the 5' sequence of the UGT1*1 exon 1 and the position of the primers with respect to the UGT gene.

### Example

We have examined the variation in the serum total bilirubin (STB) concentration in a representative group of the Eastern Scottish population (drug-free, alcohol-free non-smokers) in relation to genotype at the UDP-glucuronosyltransferase subfamily 1 (UGT1) locus. Subjects with the 77/7 genotype in this population have a significantly higher STB than those with 6/7 or 6/6 genotypes. Of 14 control subjects who underwent a 24 hour fast to establish whether they had Gilbert Syndrome (GS), only 7/77 subjects had GS. In addition, one confirmed GS patient, two recurrent jaundice patients and 9 clinically diagnosed GS patients had the 7/7 genotype. Segregation of the 7/7 genotype with elevated STB concentration has also been demonstrated in a family of 4 Gilbert members. This incidence of the 7/7 genotype in the population is 10-13%. Here, we demonstrate a correlation between variation in the human STB concentration and genotype at a TATA sequence upstream of the UGT1*1 exon 1 and that the 7/7 genotype is diagnostic for GS.

The inheritance of GS has been described as autosomal dominant or autosomal dominant with incomplete penetrance based on biochemical analysis⁶. More recent reports have suggested that the mildly affected (Gilbert) members of families in which CN type 2 (CN-2) occurs are heterozygous for mutations in the UDI³-glucuronosyltransferase subfamily 1 (UGT1) gene which cause CN-2 in the homozygous state. The inheritance of GS in these families is autosomal dominant while CN-2 is autosomal recessive⁷⁻¹¹. However, the incidence of CN-2 in the population is very rare and the frequency of alleles causing CN-2 would not be sufficient to explain the population incidence of GS.

An abstract by Bosma et al¹² suggested a correlation between homozygosity for a 2bp insertion in the TATA box upstream of UGT1*1 exon 1 and GS (no mutations were found in the coding sequence of the UGT1*1 gene). In this report we demonstrate that the primary genetic factor contributing to the variation in the serum total bilirubin (STB) concentration in the Eastern Scottish population is the sequence variation reported by Bosma et al¹². In addition, we show that the 7/77 genotype is associated with GS and occurs in 10-13% of the population.

### Methods

### Patients and Controls

Whole blood (3ml) was collected into EDTA(K3) Vacutainer tubes (Becton Dickinson) from one confirmed male Gilbert patient (diagnosed following a 48 hour restricted diet¹³), two female patients with recurrent jaundice/associated elevated STB (29-42 µmol/l) and 9 (1 female, 8 male) clinically diagnosed GS subjects (persistent elevation of the STB amidst normal liver function tests.) The patients were aged 22-45 years.

77 non-smoking residents selected at random from the Tayside/Fife region of Scotland (39 females aged 19-58 years, mean 32.41± 10.94; 38 males aged 23-57, means 35.58 ± 9.04) participated in this study. Whole blood (9ml) was collected 8-10am) into EDTA(K3) Vacutainer tubes (Becton Dickinson) for DNA extraction and SST Vacutainer tubes (Becton Dickinson) for biochemical investigations. The subjects had not taken any medication or alcohol in the previous 5-7 days and had fasted overnight (12 hours). 14 controls subsequently underwent further biochemical tests (following a 3 day abstinence from alcohol) before and after a 24 hour 400-calorie diet¹⁴ to determine if they had GS. All patients/controls were fully informed of the study and gave consent for their blood to be used in this study.

### Biochemistry and DNA Extraction

The following biochemical tests were performed on control blood samples; alanine aminostransferase, albumin, alkaline phosphatase, amylase, STB, cholesterol, creatinine, creatine kinase, free thyroxine, gamma-glutamyl-transferase, glucose, HDL-cholesterol, HDL-cholesterol/total cholesterol, iron, lactate dehydrogenase, percentage of saturated transferrin (PSAT), proteins, serum angiotensin converting enzyme, thyroid stimulating hormone, transferrin, triglycerides, urate, urea. 14 controls also had pre- and post-fasting (24 hour) alanine aminostransferase, albumin, alkaline phosphatase, STB and urate measured. DNA was prepared using the Nucleon II Genomic DNA Extraction Kit (Scotlab) according to manufacturer's instructions.

### Genotyping

### Polymerase Chain Reaction

Primer pairs A/B (A, 5'-AAGTGAACTCCCTGCTACCTT-3'; B, 5'-CCACTGGGATCAACAGTATCT-3') or C/D (C,5'-GTCACGTGACACAGTCAAAC-3';D, 5'-TTTGCTCCTGCCAGAGGTT-3') flanking the TATA box sequence upstream of the UGTI*1 exon 1 were used to amplify fragments of 253-255bp and 98-100bp, respectively. Amplifications (50µl) were performed in 0.2mM of each deoxynucleoside triphosphate (dATP, dCTP, dGTP, dTTP), 50mM KCI, 10mM Tris.HCl (pH 9.0 at 25°C), 0.1% Triton X-100, 1.5mM MgCl₂, 0.25µM of each primer, 1 Unit of Taq Polymerase (Promega) and human DNA (0.25-0.5µg). The polymerase chain reaction (PCR) conditions using the Perkin-Elmer Cetus DNA Thermal Cycler were: 95°C 5 min followed by 30 cycles of 95° 30 sec, 58°C 40 sec, 72°C40 sec.

### Direct Sequencing

Amplification was confirmed prior to direct sequencing by agarose gel electrophoresis. Sequencing was performed using [α-³⁵S]-dATP (NEN Dupont) with the USB Sequenase™ PCR Product Sequencing Kit according to manufacturer's instructions. Sequenced products were resolved on 6% denaturing polyacrylamide gels. The dried gels were exposed overnight to autoradiographic film prior to developing.

### Radioactive PCR

Amplification was performed as above using primer pair C/D except that 2.5 pmol of primer C was radioactively 5' end-labelled with 2.5µCi of [γ-³²P]-ATP (NEN Dupont) prior to amplification. Products were resolved on 6% denaturing polyacrylamide gels and the wet gels exposed to autoradiographic film (-70°C 15 min) and the autoradiographs developed.

### Statistics

A t-test was used to determine if there was a significant age difference between males and females. χ² analysis was used to assess any difference in the distribution of the 6/6, 6/7 and 7/7 genotypes in males and females and also to determine if the 7/7 subjects from the 24 hour fasted group had STB elevated into the range diagnostic for GS¹⁴. An analysis of variance was performed to compare mean STB in males and females within each genotype group. A non-parametric test, the Mann-Whitney U-Wilcoxon Rank Sum W Test was used to determine whether there was a significant difference in mean STB between males and females (irrespective of genotype). Correlations and significance tests were performed for STB versus PSAT and STB versus iron. A probability (p) of 〈 0.05 was accepted as significant.

### Results

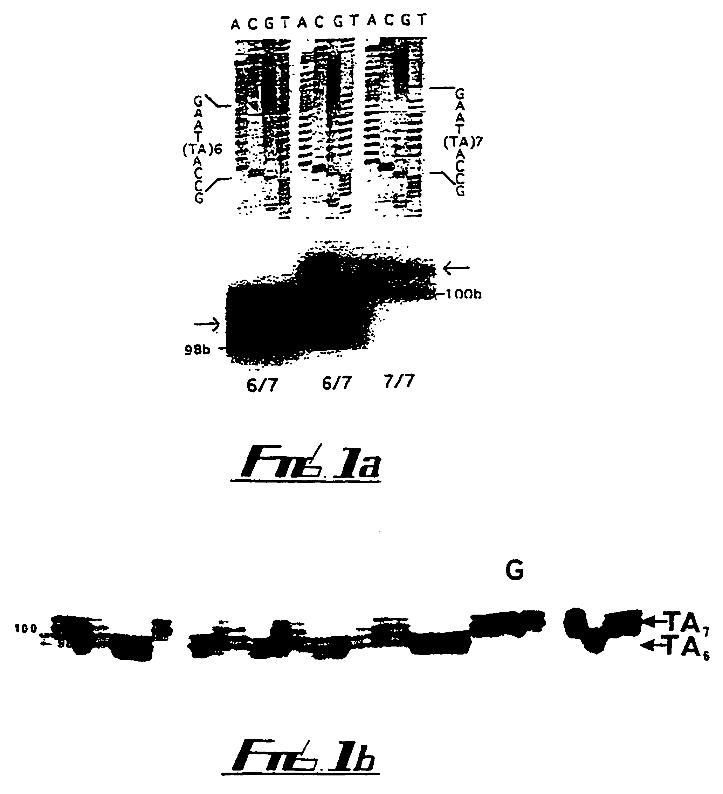

In Figure 1 a photographic representation of the sense DNA sequences obtained by PCR/direct sequencing of DNA samples having the genotypes 6/6, 6/7 and 7/7 is shown. The common allele, (TA)₆TAA, is denoted by "6" while the rarer allele, (TA)₇TAA, is denoted by "7". Below each sequence is an overexposed photographic representation of the 98 to 100bp resolved fragments amplified using primer pair C/D which flank the TATA sequence upstream of the UGT1*1 exon 1. The additional fragments of 99 and 101 bases are thought to be artifacts of the PCR process where there is non specified addition of an extra nucleotide to the 3' end of the amplified product²¹. Figures 1b illustrates results after testing a range of unknown individuals.

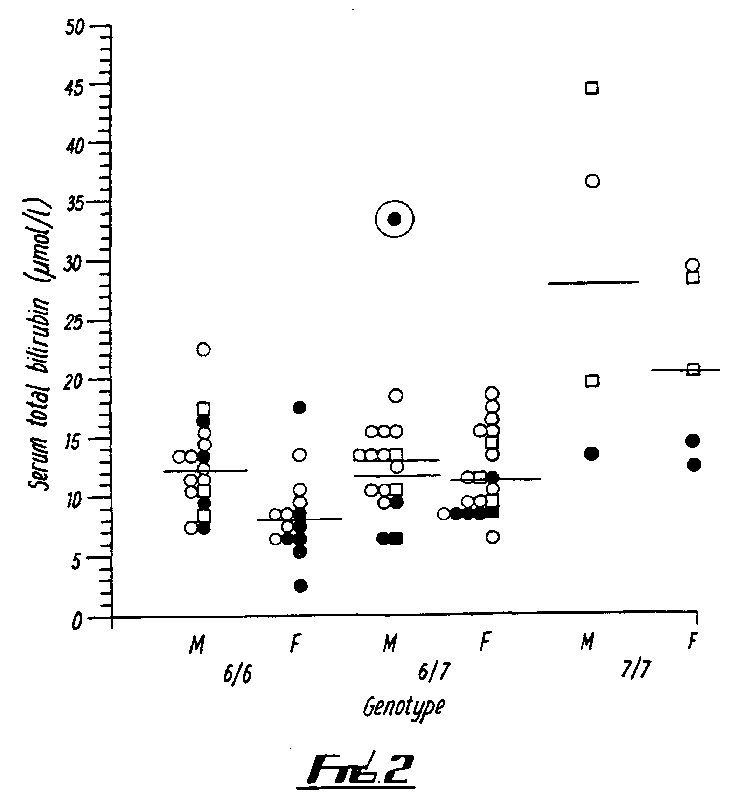

In Figure 2 males (M) and females (F) are plotted separately. Each circle/square represents the result of a single control subject. The squares indicate the 14 controls who also underwent the 24 hour restricted diet (see Methods). The filled circles/squares represent those who had a lower than normal PSAT (≤ 22%) while the half-tone circles represent those who had a higher than normal PSAT (≥ 55%). The mean STB concentrations (indicated by the horizontal lines) for males were 13.24 ± 3.88 (6/6), 13.94 ± 6.1 (6/7) including control h or 12.69 ± 3.34 excluding control h, 29 ± 14.45 (7/7) and for females were 9 ± 3.62 (6/6), 12.2 ± 3.53 (6/7), 21.6 ± 7.8 7/7). The encircled result is from control h (discussed in the text).

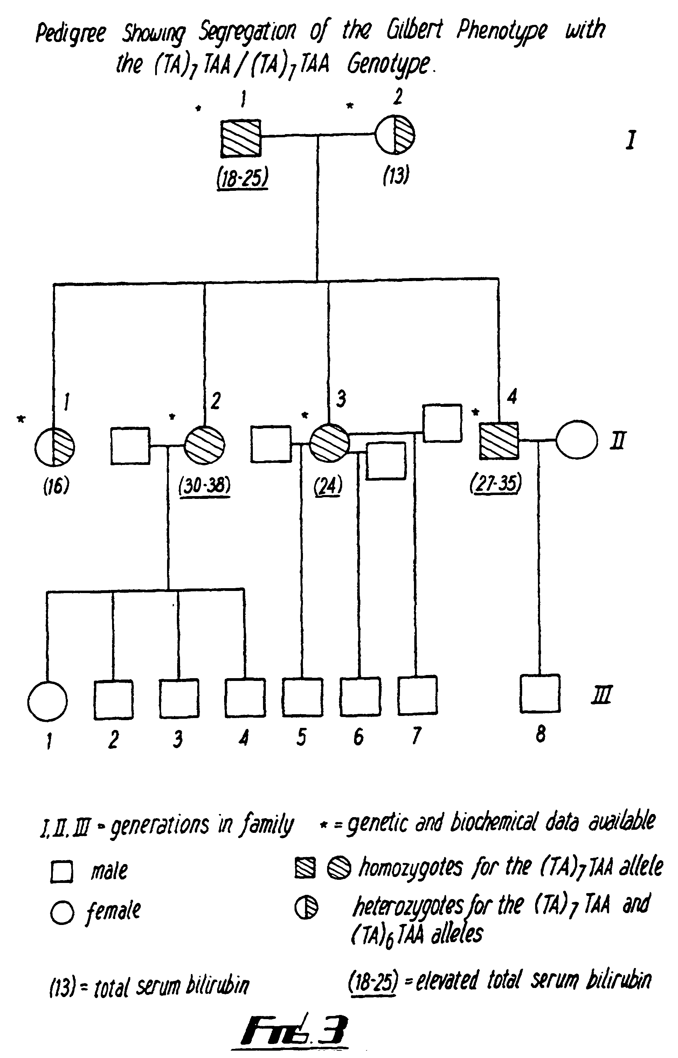

In Figure 3 males and females are represented by squares and circles, respectively. Filled and half-filled circles/squares indicate the genotypes 7/7 and 6/7, respectively. The numbers in parentheses below each member of the pedigree are the STB concentrations measured after a 15 hour fast and 7 day abstinence from alcohol. All family members were non smokers who were not taking any medication when the biochemical tests were performed. Elevated STB are underlined. Individual members of each generation (I or II) are denoted by the numbers 1-4 above each circle/square. Generation III have not yet been tested.

There was no significant age difference between males and females (t = -1.38, p = 0.17). Genotypes were determined initially by amplification/sequencing and later by the radioactive PCR approach. Individuals homozygous for the common allele, hetrozygous or homozygous for the rarer allele have the genotypes 6/6, 6/7 and 7/7, respective. 12 DNA samples (2 of 6/6, 3 of 6/7 and 4 of 7/7) were analysed by both methods and genotype results were identical (see Figure 1).

Genotype frequencies in male controls were 6/6 (44.74%, 6/7 (44.74%), 7/7 (10.52%) and in female controls were 6/6 (35.9%), 6/7 (51.3%), 7/7 (12.8%). There was no significant difference between the genotype proportions in the two groups (χ² = 0.6 at 2 df, p = 0.7). Control h (encircled in Figure 2) had a STB which was 2.4 SD above the mean STB for that group (mean calculated including control h). The results for control h were repeatable and he is currently being investigated to exclude haemochromatosis. Comparison of mean STB in males and females revealed that females have a significantly lower concentration than males (p = 0.031 including control h; p + 0.0458 excluding control h). There was a strong correlation between genotype and mean STB concentration within the control group (p 〈 0.001) irrespective of whether control h was included and there was a significant difference in mean STB between males and females of the same genotype (p 〈 0.05) irrespective of whether control h was included (see Figure 2). All patients studied had the 7/77 genotype.

Correlations between STB/PSAT (r = 0.4113, p = 0.001) (see Figure 2) and STB/iron females (p = 0.001) than males (p = 0.01) but when control h is excluded there was no significant correlation in males.

The STB concentrations of control who underwent the 24 hour restricted diet (see Methods) are shown in Table 1. The normal fasting response is a small rise in the base-line STB (not exceeding a final concentration of 25µmol/l) most of which is unconjugated while GS patients have a lone biochemical feature a raised STB (〉25µmol/l but 〈50µmol/l) most of which is unconjugated¹⁴. The 6/6 and 6/7 controls had post-fasting STB of ≤23µmol/l while all 7/77 controls were ≥31µmol/l. Other liver function tests were within acceptable ranges for the age and sex of the subjects. The 7/77 genotype correlates with a fasted STB (24 hour) within the range diagnostic for GS¹⁴ (p 〈 0.01) (see Table 1). In addition, the 7/7 genotype segregates with elevated STB concentration in a family with 4 GS members (Figures 3).

Table 1 shows a comparison of the UGT1*1 exon 1 genotype with elevation in the serum total bilirubin after a 24 hour 400-calorie restricted diet¹⁴.

An elevation of the fasting STB to a final concentration in the range 25-50µmol/l is considered to be diagnostic for GS¹⁴. The 7/7 subject denoted by * has a fasting and non-fasting STB of 〉 50µmol/l but this value is within a range considered by others to conform to a diagnosis of GS⁷⁻¹¹.

**Table 1**

| Genotype | Sex | 24 hour fast | | Fasting bilirubin >25 & <50µmol/l |
|---|---|---|---|---|
| | | Before | After | |
| | M | 8 | 17 | NO |
| 6/6 | M | 9 | 19 | NO |
| | M | 12 | 15 | NO |
| | F | 8 | 17 | NO |
| | F | 9 | 13 | NO |
| | F | 11 | 12 | NO |
| 6/7 | F | 12 | 17 | NO |
| | M | 8 | 10 | NO |
| | M | 15 | 23 | NO |
| | M | 17 | 18 | NO |
| | F | 9 | 34 | YES |
| | F | 12 | 34 | YES |
| 7/7 | M | 19 | 31 | YES |
| | M | 62 | 96 | NO* |

### Discussion

A few recent reports claim to have identified the genetic cause of GS¹⁰⁻¹². Clinical diagnosis of GS is often based on a consistent midly elevated non-fasting STB ()17 µmol/l) as the sole abnormal liver function test, intermittent jaundice or both. The diagnosis can be confirmed by elevation of the STB to 25-50µmol/l after a 24 hour 400-calorie diet¹⁴ or by elevation of the unconjugated bilirubin by 〉 90% within 48 hours of commencing a 400 calorie diet¹³.

Sato's research group recently reported the occurrence of 7 different heteroxygous missence mutations in unrelated Gilbert patients (most of the mutations have been found in the homozygous state in affected members of CN families), however, the non-fasted STB for these patients were 〉 52µmol/l (with the exception of one, 31µmol/l)^{10,12}. These non-fasted STB concentrations already exceed the diagnostic range for GS¹⁴, hence these patients have a more severe form of hyperbilirubinaemia than those studied in this report, while those in the Bosma et al¹² abstract had STB concentrations similar to those studied here.

The example herein shows that the variation in the STB levels after an overnight fast (and in the absence of exposure to known inducers of the UGT1*1 isoform in GS, such as alcoholic¹⁵ and drugs¹⁶) a representative group of the Eastern Scottish population is primarily due to (or associated with) the TATA box sequence variation reported by Bosma et al¹². In agreement with previous work females have a significantly lower mean STB concentration than males¹⁷⁻¹⁸.

Individuals with the 7/7 genotype in the population have GS (see Table 1). One of the 7/7 controls indicated in Table 1 had a non-fasting STB similar to those reported for heterozygous carriers of CN-2 mutations⁷⁻¹¹ which suggests that this subject may also be a carrier of a CN-2 mutation, alternatively, the very elevated bilirubin in this patient may be due to the coexistence of Reavon's Syndrome (characterized by a collection of abnormal biochemical results which are risk factors for coronary heart disease)¹⁹.

We have found that 10-13% of the Eastern Scottish population have the genotype associated with mild GS. None of the Gilbert subjects from the control population were aware that they had an underlying metabolic defect in glucuronidation with testifies to its benign nature. Three 7/7 controls had STB concentrations comparable to mean levels observed in heterozygotes, however, they also had a lower than normal PSAT (≤22%) (see Figure 2). The observed correlation between STB and PSAT (p = 0.001) (Figure 2) and STB and iron (females p = 0.001 and males p = 0.01 including control h) indicates that other genetic and environmental factors affecting the serum PSAT and iron values will in turn affect the STB concentration.

From the data presented here and previous reports it seems clear that there are mild and more severe forms of GS. The milder form (fasted STB 25-50µmol/l) is either caused by (or is associated with) a homozygous 2bp insertion at the TATA sequence upstream of the UGT1*1 exon 1 (autosomal recessive inheritance) while the rarer more severe dominantly inherited forms identified to date⁷⁻¹¹ (non-fasted STB 〉 50µmol/l are due to heterozygosity for a mutation in the coding region of the UGT1*1 gene which in its homozygous state causes CN-2. The particular genetic abnormality causing GS in a patient will have implications for genetic counselling as the dominantly inherited form of two GS patients could result in offspring with CN-2, whereas the recessive form in one or both GS patients would have less serious implications. It is important to discriminate between the two forms and provide suitable genetic counselling for such couples. The rapid DNA test presented here (less than 1 day for extracted DNA) carried out in addition to biochemical tests following a 12 hour overnight fast (without prior alcohol or drug intake would permit such a diagnosis. The compliance rate for the current 24 and 48 hour restricted diet tests for GS¹³⁻¹⁴ is debatable and hence the overnight fast has obvious advantages and only one blood sample or a buccal smear is required (for genetic and biochemical analysis) in contrast to the 2-3 blood samplings required for the 24 and 48 hour tests. This approach to GS testing would be cost effective in terms of fewer patient return visits to clinics and in identifying couples at risk of having children with CN-2.

In addition, the recent finding of an increased bioactivation of acetominophen (a commonly used analgesic which is eliminated primarily by glucuronidation) in GS patients indicates the greater potential for drug toxicity in these patients if administered drugs which are also conjugated by UGT1 isoforms³. In fact, ethinylestradiol (EE2) has recently been shown to be primarily glucuronidated by the UGT1*1 isoform in man²⁰ and hence this could have implications for female Gilbert patients taking the oral contraceptive who are then more predisposed to developing jaundice.

The tests outlined herein have obvious implications for setting up drug trials in understanding unusual results in ruling out individuals who may be adversely affected by the drugs or in positively choosing these individuals to determine the effects of particular drugs on hyperbilirubinaemia.

### References

1 Fevery, J. Pathogenesis of Gilbert Syndrome. Eur. *J. Clin. Invest.* 1981;11; 417-418.
2. Watson, K.J.R. and Gollan, J.L. Gilbert's Syndrome. *Bailliere's Clinical Gastroenterology* 1989; 3: 337-355.
3. De Morais, S.M.F., Uetrecht, J.P. and Wells, P.G. Decreased glucuronidation and increased bioactivation of acetaminophen in Gilbert's Syndrome. *Gastroenterology* 1992; 102: 577-586.
4. Carulli, N., Ponz de Leon, M., Mauro, E., Manenti, F and Ferrari, A. Alteration of drug metabolism in Gilbert's Syndrome. Gut 1976; 17: 581-587.
5. Macklon, A.F., Savage, R.L. and Rawlins, M.D. Gilbert Syndrome and.drug metabolism. *Clin*. *Pharmacokinetics* 1979; 4: 223-232.
6. Thompson, R.PH.H. Genetic transmission of Gilbert's Syndrome in "Familial Hyperbilirubinaemia", (Ed. L. okoliosanyi), *John Wiley* & *Sons* Ltd; 91-97.
7. Gollan, J.L. Huang, S.N., Billing, B. and Sherlock, S. Prolonged survival in three brothers with severe type 2 Crigler-Najjar Syndrome. *Gastroenterology* 1975; 68: 1543-1555.
8. Moghrabi, N., Clarke, D.J., Boxer, M. and Burchell, B. Identification of an A-to-G missence mutation in exon 2 of the UGT1 gene complex that causes Crigler-Najjar Syndrome type 2. *Genomics* 1993; 18: 171-173.
9. Moghrabi, N.N. Molecular Genetic Analysis of the Human Phenol and Bilirubin UDP-Glucuronosyltransferase Gene Complex and Associated Disease Syndromes. *PhD thesis* 1994, University of Dundee, Dundee, Scotland.
10. Aono, S., Adachi, Y., Uyama, E., Yamada, Y., Keino, H., Nanno, T., Koiwai, O. and Sato, H. Analysis of genes for bilirubin UDP-glucuronosyltransferase in Gilbert's Syndrome, Lancet 1995; 345: 958-959.
11. Koiwai, O., Nishizawa, M., Hasada, K., Aono, S., Adachi, Y., Mamiya, N. and Sato, H. Koiwai, O., Nishizawa, M., Hasada, K., Aono, S., Adachi, Y., Mamiya, N. and Sato, H. Gilbert's Syndrome is caused by a heterozygous missence mutation in the gene for bilirubin UDP-glucuronosyltransferase. *Hum. Molec. Genet.* 1995; 4: 1183-1186.
12. Bosma, P., Goldhoorn, B., Bakker, C., Out, T., Roy Chowdhury, J., Roy Chowdhury, N., Oostra, B., Lindhout, D., Michiels, J., Jansen, P., Tytgat, G. and Oude Elferink, R. Presence of an additional TA in the TATAA box of B- UGT1 correlates with Gilbert Syndrome. *Hepatology* October 1994; Abstract 680: 226A.
13. Owens, D. and Sherlock, S. Diagnosis of Gilbert's Syndrome: role of reduced calorie intake test. *Br. Med.J.* 1973; 3: 559-563.
14. Lascelles, P.T. and Donaldson, D. Calorie restriction test in "Diagnostic Function Tests in Chemical Pathology" *Kluwer Academic Publishers* 1989: 24-25.
15. Ideo, G., De Franchis, R., Del Ninno, E. and Dioguardi, N. Ethanol increases liver uridine-diphosphate-glucuronosyltransferase. *Experientia* 1971; 27: 24-25.
16. Sutherland, L.T., Ebner, T. and Burchell, B. Expression of UDP-Glucuronosyltransferases (UGT) 1 family in human liver and kidney. *Biochem. Pharmacol.* 1993; 45: 295-301.
17. Owens, D. and Evans, J. Population studies on Gilbert Syndrome. *J. Med.* Genet. 1975;12: 152-156.
18. Bailey, A., Robinson, D. and Dawson, A.M. Does Gilbert's disease? Lancet 1977; 1: 931-933.
19. Reaven, G.M. Syndrome X: 6 years later. *J. Intern. Med.* 1994; 236: 13-22.
20. Ebner. T., Remmel, R.P. and Burchell, B. Human bilirubin UDP-glucuronosyltransferase catalyses the glucuronidation of ethinylestradiol. *Molec*. *Pharmacol*. 1993; 43: 649-654.
21. Edwards, A., Hammond, H.A., Jin, L., Caskey, C.T. and Chakraborty, R. Genetic variation at five trimeric and tetrameric tandem repeat loci in four human population groups. Genomics *1992; 12:* 241-253.

## Claims

1. A method of conducting a drug trial, the method comprising screening samples from potential participants for the basis of Gilbert's Syndrome and eliminating or including potential participants in a drug trial in the knowledge of them possessing or not possessing the genetic basis of Gilbert's Syndrome, and interpreting hyperbilirubinaemia and/or jaundice occurring during the drug trial in the knowledge that certain participants have or do not have Gilbert's Syndrome.

2. A method according to claim 1 wherein the method comprises the steps of:
a) screening a sample from each potential participant in a drug trial for the genetic basis of Gilbert's Syndrome;
b) identifying participants having the genetic basis of Gilbert's Syndrome;
c) proceeding with drugs trials in the knowledge of participants possessing or not possessing the genetic basis of Gilbert's Syndrome; and
d) interpreting hyperbilirubinaemia and/or jaundice occurring during the drug trial in the knowledge that certain participants have or do not have Gilbert's Syndrome.

3. A method as claimed in claim 1 or 2 wherein the sample is chosen from blood, buccal smear or any other sample containing DNA from the potential participants.

4. A method as claimed in claim 1 further comprising the step of eliminating participants having the genetic basis of Gilbert's Syndrome from a drugs trial.

5. A method as claimed in any of the preceding claims wherein the method comprises the steps of:
a) isolating DNA from each sample;
b) amplifying the DNA inner region indicating the genetic basis for Gilbert's Syndrome;
c) isolating amplified DNA fragments; and
d) identifying individuals having the genetic basis of Gilbert's Syndrome.

6. A method as claimed in any of the preceding claims wherein the DNA is amplified using the polymerase chain reaction (PCR) using a radioactively labelled pair of nucleotide primers.

7. A method as claimed in either of claims 5 or 6 wherein the DNA region indicating the genetic basis of Gilbert's Syndrome is the gene encoding UDP-glucuronosyltransferase (UGT).

8. A method as claimed in any of claims 5 to 7 wherein the DNA to be amplified includes the region between -35 and -55 nucleotides upstream of the UGT 1*1 exon 1 as identified in Figure 4.

9. A method according to claim 8 wherein primers for amplifying DNA in the region identified are AB or CD as follows: A/B(A, 5'-AAGTGAACTCCCTGCTACCTT-3', B, 5'-CCACTGGGATCAACAGTATCT-3') or

C/D (C, 5'-GTCACGTGACACAGTCAAAC-3',

D, 5'-TTTGCTCCTGCCAGAGGTT-3').

10. A method of conducting a drug trial, the method comprising screening a sample from each participant of a drug trial for the genetic basis of Gilbert's Syndrome and identifying participants having the genetic basis of Gilbert's Syndrome, carrying out said drug trial and detecting serum total bilirubin such that it is possible to detect whether high levels of serum total bilirubin in a participant of said drug trial is caused by the drug or Gilbert's Syndrome.

## Patentansprüche

1. Verfahren zum Durchführen einer Arzneimitteluntersuchung, wobei das Verfahren umfasst: Screenen von Proben von potentiellen Teilnehmern im Hinblick auf die Basis für das Gilbert-Syndrom, und Eliminieren oder Einschließen potentieller Teilnehmer von einer/in eine Arzneimitteluntersuchung im Wissen, dass sie die genetische Basis für das Gilbert-Syndrom besitzen oder nicht besitzen, und Interpretieren der während des Arzneimittelversuchs auftretenden Hyperbilirubinämie und/oder Gelbsucht, im Wissen, das gewisse Teilnehmer das Gilbert-Syndrom haben oder nicht haben.

2. Verfahren gemäß Anspruch 1, wobei das Verfahren Folgende Schritte umfasst:
a) Screenen einer Probe von jedem potentiellen Teilnehmer an einer Arzneimitteluntersuchung im Hinblick auf die genetische Basis für das Gilbert-Syndrom;
b) Identifizieren der Teilnehmer, die die genetische Basis für das Gilbert-Syndrom haben;
c) Durchführen von Arzneimitteluntersuchungen im Wissen, dass die Teilnehmer die genetische Basis für das Gilbert-Syndrom besitzen oder nicht besitzen; und
d) Interpretieren der Hyperbilirubinämie und/oder Gelbsucht, die während des Arzneimittelversuchs auftreten, im Wissen, dass gewisse Teilnehmer das Gilbert-Syndrom haben oder nicht haben.

3. Verfahren gemäß Anspruch 1 oder 2, wobei die Probe aus Blut, Wangenabstrich und anderen Proben ausgewählt wird, die DNA der potentiellen Teilnehmer enthalten.

4. Verfahren gemäß Anspruch 1, umfassend des Weiteren den Schritt des Eliminierens von Teilnehmern, die die genetische Basis für das Gilbert-Syndrom aufweisen, aus einer Arzneimitteluntersuchung.

5. Verfahren gemäß einem der vorhergehenden Ansprüche, wobei das Verfahren folgende Schritte umfasst:
a) Isolieren von DNA aus jeder Probe;
b) Amplifizieren der inneren DNA-Region, die die genetische Basis für das Gilbert-Syndrom anzeigt;
c) Isolieren von amplifizierten DNA-Fragmenten; und
d) Identifizieren von Individuen, die die genetische Basis für das Gilbert-Syndrom aufweisen.

6. Verfahren gemäß einem der vorhergehenden Ansprüche, wobei die DNA unter Verwendung der Polymerase-Kettenreaktion (PKR) mit Hilfe eines radioaktiv markierten Paars von Nukleotidprimern amplifiziert wird.

7. verfahren gemäß einem der Ansprüche 5 oder 6, wobei die DNA-Region, die die genetische Basis für das Gilbert-Syndrom anzeigt, die genkodierende UDP-Glucoronosyltransferase (UGT) ist.

8. Verfahren gemäß einem der Ansprüche 5 bis 7, wobei die zu amplifizierende DNA die Region zwischen -35 und -55 Nukleotiden oberhalb vom UGT 1*1 Exon 1, wie in Figur 4 identifiziert, einschließt.

9. Verfahren gemäß Anspruch 8, wobei die Primer zum Amplifizieren der DNA in der identifizierten Region AB oder CD wie folgt sind: A/B(A,5'AAGTGAACTCCCTGCTACCTT-3', B, 5'-CCACTGGGATCAACAGTATCT-3') oder

C/D (C, 5′-GTCACGTGACACAGTCAAAC-3′,

D, 5'-TTTGCTCCTGCCAGAGGTT-3').

10. Verfahren zum Durchführen einer Arzneimitteluntersuchung, wobei das Verfahren umfasst: Screenen einer Probe von jedem Teilnehmer an einer Arzneimitteluntersuchung im Hinblick auf die genetische Basis für das Gilbert-Syndrom und Identifizieren der Teilnehmer, die die genetische Basis für das Gilbert-Syndrom aufweisen, Durchführen des Arzneimittelversuchs und Bestimmen des Gesamtserumbilirubins derart, dass es möglich ist, zu bestimmen, ob hohe Gehalte an Gesamtserumbilirubin bei einem Teilnehmer an dem Arzneimittelversuch durch das Arzneimittel oder das Gilbert-Syndrom verursacht worden sind.

## Revendications

1. Procédé pour la réalisation d'un essai de médicament, comprenant la sélection d'échantillons provenant de participants potentiels sur la base du syndrome de Gilbert et la suppression ou l'ajout de participants potentiels à un essai sur médicament en sachant qu'ils possèdent ou ne possèdent pas la base génétique du syndrome de Gilbert, et l'interprétation d'une hyperbilirubinémie et/ou d'un ictère se produisant lors de l'essai du médicament en sachant que certains participants présentent ou ne présentent pas le syndrome de Gilbert.

2. Procédé selon la revendication 1 comprenant les étapes de :
a) sélection d'un échantillon provenant de chaque participant potentiel à un essai de médicament sur la base génétique du syndrome de Gilbert ;
b) identification des participants présentant la base génétique du syndrome de Gilbert ;
c) poursuite des essais de médicaments en sachant quels participants possèdent ou ne possèdent pas la base génétique du syndrome de Gilbert ; et
d) interprétation d'une hyperbilirubinémie et/ou d'un ictère se produisant lors de l'essai du médicament en sachant que certains participants présentent ou ne présentent pas le syndrome de Gilbert.

3. Procédé selon les revendications 1 ou 2 dans lequel l'échantillon est sélectionné parmi du sang, un frottis buccal ou tout autre échantillon contenant de l'ADN des participants potentiels.

4. Procédé selon la revendication 1 comprenant en outre l'étape d'élimination des participants présentant la base génétique du syndrome de Gilbert d'un essai de médicaments.

5. Procédé selon l'une quelconque des revendications précédentes, comprenant les étapes de :
a) isolation de l'ADN de chaque échantillon ;
b) amplification de la région interne de l'ADN indiquant la base génétique du syndrome de Gilbert ;
c) isolation des fragments d'ADN amplifiés ; et
d) identification des individus présentant la base génétique du syndrome de Gilbert.

6. Procédé selon l'une quelconque des revendications précédentes dans lequel l'ADN est amplifié par réaction de polymérisation en chaîne (PCR) en utilisant une paire marquée radioactivement d'amorces nucléotidiques.

7. Procédé selon l'une quelconque des revendications 5 ou 6 dans lequel la région d'ADN indiquant la base génétique du syndrome de Gilbert est le gène codant la UDP-glucuronosyltransférase (UGT).

8. Procédé selon l'une quelconque des revendications 5 à 7 dans lequel l'ADN devant être amplifié inclut les régions entre les nucléotides -35 et -55 en amont de l'exon 1 UGT 1*1 tel qu'identifié sur la figure 4.

9. Procédé selon la revendication 8 dans lequel les amorces pour amplifier l'ADN dans la région identifiée sont AB ou CD comme suit : A/B(A, 5'-AAGTGAACTCCCTGCTACCTT-3', B, 5'-CCACTGGGATCAACAGTATCT-3') or

C/D (C, 5'-GTCACGTGACACAGTCAAAC-3',

D, 5'-TTTGCTCCTGCCAGAGGTT-3').

10. Procédé pour la réalisation d'un essai de médicament, comprenant la sélection d'un échantillon de chaque participant d'un essai de médicament sur base génétique du syndrome de Gilbert et l'identification des participants présentant la base génétique du syndrome de Gilbert, la réalisation dudit essai de médicament et la détection de la bilirubine sérique totale de sorte qu'il soit possible de détecter si des niveaux élevés de bilirubine sérique totale chez un participant audit essai de médicament sont causés par le médicament ou le syndrome de Gilbert.
